# EUROPEAN PATENT SPECIFICATION

(11) **EP 1 736 177 B1**
(45) Date of publication and mention of the grant of the patent: **16.06.2010**
(21) Application number: 04724027.0
(22) Date of filing: 29.03.2004
(51) Int. Cl.: A61L 9/02, A61L 9/12

(54) **METHOD AND DEVICE FOR THE EVAPORATION OF VOLATILE SUBSTANCES**
VERFAHREN UND VORRICHTUNG ZUR VERDÜNSTUNG VON FLÜCHTIGEN SUBSTANZEN
PROCEDE ET DISPOSITIF D'EVAPORATION DE SUBSTANCES VOLATILES

(43) Date of publication of application: 27.12.2006
(73) Proprietor: Zobele España, S.A., 08290 Cerdanyola del Vallés (ES)
(72) Inventor: Caserta, Andrea, c/o Parc Tecnologic del Valles, 08290 Cerdanyola del Valles (ES); Garcia Fabrega, R., c/o Parc Tecnologic del Valles, 08290 Cerdanyola del Valles (ES)
(74) Representative: Carpintero Lopez, Francisco
(86) International application number: PCT/ES2004/000139
(87) International publication number: WO 2005/092399

(56) References cited:
- EP-A1- 0 942 648
- ES-A1- 2 137 111
- ES-A1- 2 185 490
- US-A- 4 968 487

## Description

### OBJECT OF THE INVENTION

The present invention is related with a method and a device for the evaporation of volatile substances such as aromatic substances and/or insecticides, in which the evaporation is done by electrical heating means that raise the temperature of a wick impregnated with the substance to be evaporated.

In the present invention the degree of evaporation of the volatile substance can also be controlled.

### BACKGROUND OF THE INVENTION

Devices to evaporate volatile substances in a liquid state are well known that consist of a wick of which one end is in contact with a volatile substance in a bottle, such that the substance rises by capillarity through the wick, which is made of a porous material, until this becomes totally impregnated.

Conventionally, these devices include heating means, such as PTC electrical resistances, aimed at heating the upper portion of the wick that facilitates evaporation of the substance that dissipates to the exterior of the device in the form of vapor.

Some of these pieces of equipment are fitted with devices that permit the degree of evaporation of the product to be controlled, which are generally based on modifying the intensity at which the wick is heated. The need to control the degree of evaporation tends to complicate the design and manufacture of the device since several interconnecting parts are required, making the manufacturing process more expensive, which is a key factor in these products since the low production costs are the key to their profitability.

Some known devices of this kind use heating means with a toroidal configuration such that one end of the wick is lodged in the central orifice, permitting uniform heating of the whole perimeter of the wick in the area of influence of the heating element. Although this toroidal shape of the heating element is effective from an operative perspective, the device as a whole increases in width and, therefore, size and this is undesirable from manufacturing and sales' perspectives.

Some examples of this type of device can be found in the patents US-4.739.928, EP-1.270.022, US-6.659.301.

Other prior-art devices for evaporation of volatile substances having toroidal-shaped heating means are described in the following patents: ES-2.185.490, ES-2.137.111, US-4.968.487.

The European patent application EP-1.064.957, describes a device of this type which is also provided with heating means of toroidal configuration, in which regulation of the degree of evaporation is achieved by moving a cap vertically.

It is also known from European application EP-1.283:062, an electric evaporator provided with a wick and a draught regulator.

### DESCRIPTION OF THE INVENTION

The present invention refers to a method and a device for the evaporation of volatile substances that optimally exploits the heat energy generated. The invention is as defined in the independent claims.

In addition to the afore-mentioned advantages, with the said invention the user can control at will the degree of evaporation of the substance and achieves this using a single element that maximally simplifies the manufacturing process and the cost of the product.

Therefore, one of the aspects of the invention refers to a device for the evaporation of volatile substances that includes a wick through which the said substance travels upwards by capillarity, which is influenced by heating means that facilitate this evaporation. The device has a pipe with open ends that contains part of the wick, with a space around the wick between this and the sides of the tube. The pipe has at least one opening in the side that controls the degree of exposure of the wick to the focus of heat produced by the heating means.

In this way, part of the heat generated by the heating means passes to the chamber inside the pipe that contains part of the wick. The pipe reduces the volume of space surrounding the wick, thus less heat energy is required to obtain the degree of evaporation desired. This reduced volume facilitates a "chimney effect", i.e. an increased rate of release of the evaporated fragrance, which causes increased diffusion of the product.

The invention incorporates means that enable the user to control the degree of evaporation of the substance by controlling the degree of influence of heat on the wick.

Another aspect of the invention refers to a method to evaporate volatile substances that includes putting a wick impregnated with the volatile substance to be evaporated, under the influence of a heat source that consists in inserting part of the wick into a small-volumed chamber and introducing hot air into the chamber. Reduced volume refers to a chamber with a volume slightly larger than that of the portion of wick inside it, such that there is a narrow space around the wick between this and the sides of the pipe through which the hot air can rise.

This chamber is defined by tubular pipe, open at both ends, which has at least one lateral opening such that in the method of the invention hot air produced by the heating source is introduced through this lateral opening of the pipe and spreads throughout the interior of the chamber remaining in close proximity to the wick while it rises up through the pipe.

In the method, the amount of hot air introduced in the chamber can be controlled in order to modify the degree of evaporation.

In a preferred option of the method, the amount of air is controlled by moving the position of the pipe relative to the heat source so that the opening faces the heat source to a greater or lesser degree thus resulting in a greater or lesser transfer of radiation and convection to the inside of the pipe and to the surface of the wick exposed to the heat.

### DESCRIPTION OF THE DRAWINGS

Complementary to the description given here this is accompanied, as an integral part of this description, by a set of diagrams, of an illustrative and not restrictive nature, aimed at helping to clarify the characteristics of the invention in accordance with an example of a practical application of the said invention. The said diagrams represent the following:
Figure 1.- Figure 1a shows a view, in perspective, of the evaporation device without the front part of its casing, in which the pipe is in the position corresponding to minimum evaporation, while figure 1 b shows a similar representation of the previous figure but in which the pipe is in the position corresponding to maximum evaporation.
Figure 2.- Figure 2a shows a side view of the evaporation device without the front part of its casing, in which the pipe is in the maximum evaporation position while figure 2 b shows a similar representation to that of the previous figure but in which the pipe is in the minimum evaporation position. In both figures, the heat generated by the heating means is represented by three black arrows.
Figure 3- shows a figure, in perspective, of the wick, the pipe in which it is inserted and the heating means, where the direction of heat radiated is represented by arrows.
Figure 4.- shows a similar figure to the previous one showing a frontal view of the same parts. The figure reveals an improved exit of the convective flow (a smaller cross-sectional area results in increased exit speed and, therefore, greater range).

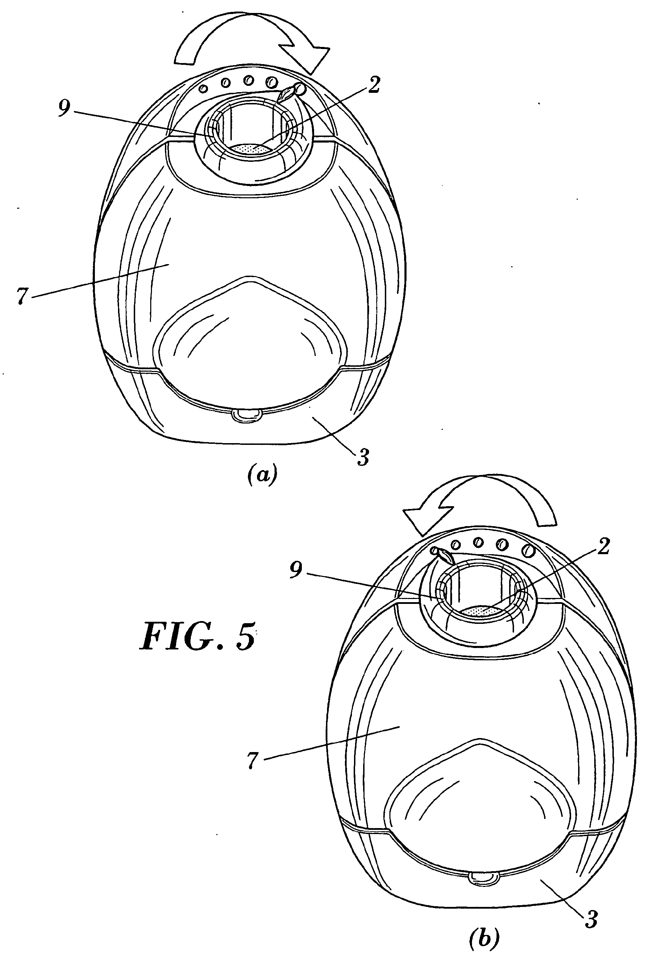

Figure 5 .- shows both views of the evaporation device, in perspective, with its graduated evaporation scale of which Figure 5 a shows the device in the maximum evaporation position and figure 5b the device in the minimum evaporation position.

### PREFERABLE EMBODIMENT OF THE INVENTION

In the light of the figures described it can be observed how in one of the possible embodiments of the invention, device (1) includes a wick (2) the lower end of which is submerged inside a bottle (3) that contains the substance to be evaporated in a liquid state, which impregnates the whole wick (2). The heating means (4) comprised for example of a cemented resistance are located near the upper part of the wick (2) heating the said region.

The device (1) of the invention comprises a cylindrical pipe (5) which contains a portion of the wick (2).

The pipe (5) has an opening (6) in the side, and is fitted into a casing (7) that forms part of the device (1), on which it can rotate in one plane, i.e. it is free to rotate on its axis but cannot be displaced vertically. The resistor (4) is firmly attached to the casing (7), thus rotation of the pipe (5) changes the position of the opening (6) relative to the resistance (4) and, therefore, changes the heat flow transmitted to the wick in the pipe (5).

In another preferable embodiment (not represented here), the device can have two small resistancesresistors (4) situated at each side of the pipe (5) which, in turn, would have two openings, which could reduce even further the dimensions of the device and produce a more uniform heating of the wick since the hot air would affect opposite sides of the device.

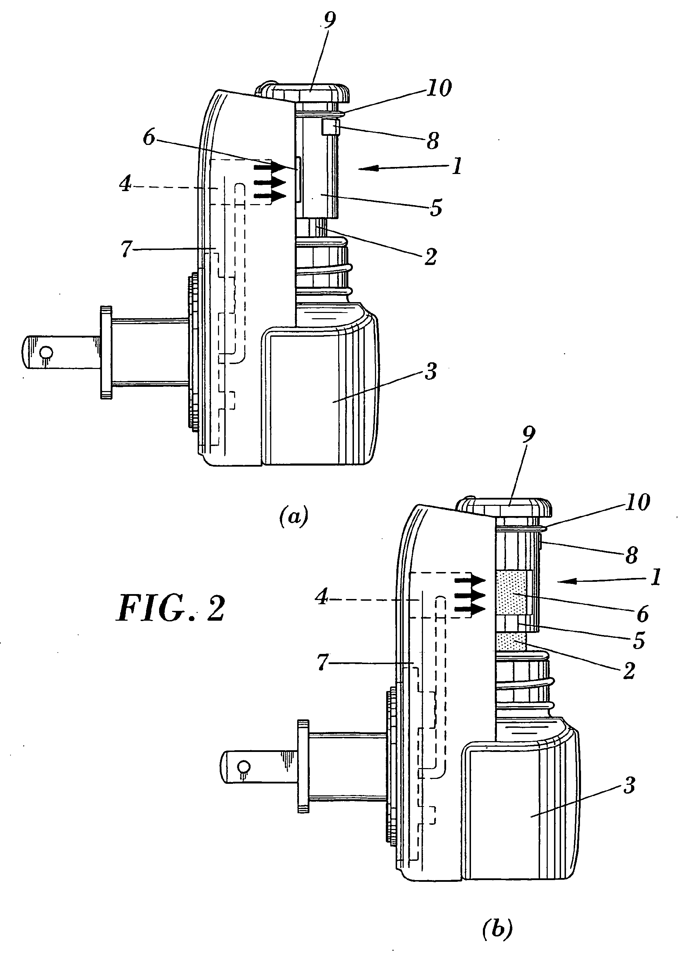

The resistors used are flat in order to occupy the smallest possible space inside the casing (7), as can be seen in Figure 2. This same Figure 2 shows how the resistanceresistor (4) is located in the same plane, i.e. at the same height as the opening (6), so that the heat generated by it reaches the wick (2) more directly and more heat enters the pipe (5). In the different positions of the pipe (5), the position and distance of the opening (6) are modified (6) relative to the resistor (4), which, in turn, alters the surface of the wick that directly receives the heat from the resistor (4).

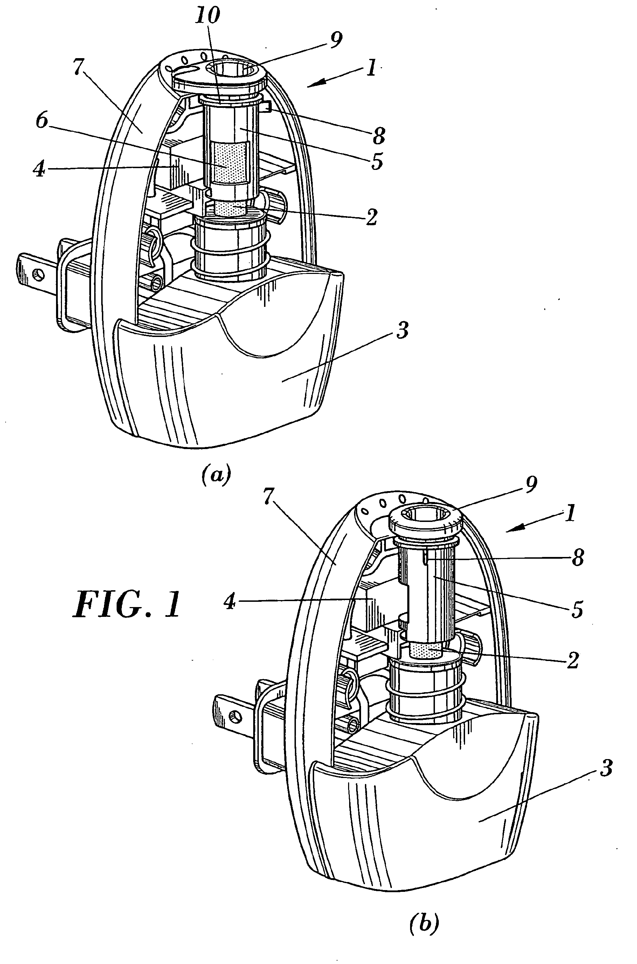

In this way, two extreme positions are established in the pipe position (5), these are limited by the contact of a flange (8) attached to the pipe (5), with catches to limit rotation of the pipe on the inside of the casing (7). Therefore, a first minimum evaporation end position is defined, as can be observed in figures 1 a and figure 2 b, in which the opening (6) is not facing the resistor (4) and, therefore, the entrance of hot air through the opening (6) is minimal or practically nil.

In a second extreme position of maximum evaporation, represented in figures 1 b and 2 a, the entire length of the opening (6) is opposite the resistor (4), thus the intake of hot air into the pipe (5) through the opening (6) is maximum.

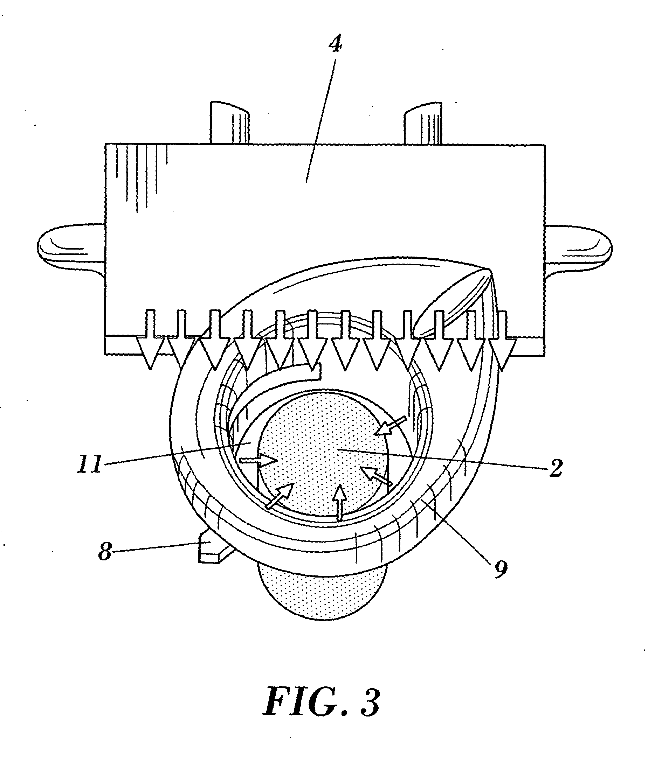

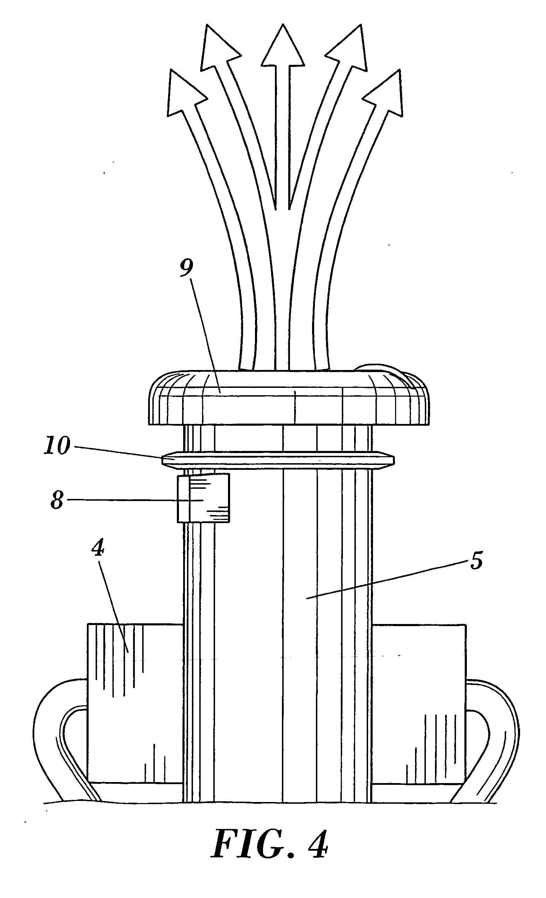

Figure 3 shows how the hot air that enters the pipe (5), is distributed radially around the length of the wick (2) as it rises through the perimetric space (11), until it leaves the pipe as shown in Figure 4.

The upper end of the pipe (5) emerges from the upper end of the casing (1) forming an annular protuberance (9) facilitating manual handling by the user. For this purpose, the pipe (5) has a lip on its perimeter (10) that overlaps an internal part of the casing (1), which can be found between this lip (10) and the annular protuberance (9), preventing displacement of the pipe (5) vertically but permitting it to rotate.

In the light of this description and set of figures, an expert in the art can understand that the description of the invention corresponds to preferential embodiments.

## Claims

1. Device for the evaporation of volatile substances comprising
heating means (4),
a wick (2) through which said substance travels upwards by capillarity, which is influenced by said heating means (4) to facilitate evaporation of said substance,
a tubular pipe (5) with open ends which contains part of the wick (2), wherein a perimetric chamber (11) around the wick is defined between said part of the wick and the tubular pipe (5), so that a chimney effect can be created in said perimetric chamber (11),
and a casing (7);
wherein said tubular pipe (5) has at least one lateral opening (6) arranged to allow hot air from said heating means (4) to enter into said perimetric chamber (11) and to rise up through the interior of the tubular pipe (5) to influence the wick (2), and wherein the heating means are fixed and the tubular pipe (5) is fitted into the casing (7) so that the tubular pipe can rotate in one plane and about its axis, and so that the tubular pipe (5) cannot be displaced vertically,
and wherein the heating means (4) and the opening (6) of the pipe are located at the same height, such that a part of the wick can face the heating means through said opening (6).

2. Device according to claim 1 **characterized in that** rotation of the pipe changes the position of the opening (6) relative to the heating means (4) so that, the amount of heat that influences the wick is varied.

3. Device according to any one of the previous claims **characterized in that** the heating means comprise, at least, an electrical resistor located close to the wick.

4. Device according to any one of the previous claims **characterized in that** the heating means comprise two resistances positioned diametrically to the pipe and **in that** the said pipe has two lateral openings.

5. Device according to claim 3 or 4, **characterized in that** the resistor or resistors define at least one flat surface.

6. Device according to any one of claims 3 to 5, **characterized in that** the resistor or resistors have a prismatic rectangular shape.

7. Device according to claim 1 **characterized in that** in the rotational displacement of the pipe, a first extreme minimum evaporation position is defined in which the opening is not facing the heating means and a second extreme maximum evaporation position in which the opening faces these heating means.

8. Device according to any one of the previous claims **characterized in that** the heating means are supported by said casing.

9. Device according to claim 8 **characterized in that** the upper end of the pipe juts out of the top of the casing forming an annular protuberance (9) that facilitates its manual movement relative to the casing (7).

10. Device according to either of claims 8 or 9 **characterized in that** the pipe has a perimetric lip and **in that** part of the casing is located between said perimetric lip and the annular protuberance (9), preventing vertical displacement of the pipe but permitting this to rotate.

11. Device according to any one of the previous claims **characterized in that** the pipe is cylindrical.

12. Device according to any one of the previous claims **characterized in that** it comprises a bottle (3) that contains an aromatic substance and/or an insecticide volatile substance, and wherein a part of the wick is inserted in said bottle.

13. Method of evaporation of volatile substances using a device according to any one of claims 1-12, said method comprising the step of enclosing a portion of a wick (2) in a small volumed-chamber (11) defined by a tubular pipe (5) with open ends, so that a narrow space is defined surrounding the wick between said tubular pipe (5) and the wick through which hot air can rise up, and in such a manner that a chimney effect can be created in said chamber, wherein hot air is introduced into said chamber through a side opening (6) provided in the tubular pipe (5) so that the hot air spreads throughout the interior of the chamber remaining in close proximity with the wick while it rises up through the pipe, and wherein the amount of air entering into said chamber (11) is controlled by rotating the tubular pipe (5) about its axis and in one plane, that is, without displacing the tubular pipe vertically, so that the distance between the side opening (6) and the heat source is modified.

## Patentansprüche

1. Vorrichtung zum Verdunsten von flüchtigen Substanzen mit:
Heizeinrichtungen (4);
einem Docht (2), durch welchen sich die Substanz durch Kapillarwirkung nach oben bewegt, wobei die Heizeinrichtungen (4) diesen zur Vereinfachung des Verdunstens der Substanz beeinflussen;
einem röhrenförmigen Rohr (5) mit offenen Enden, das einen Teil des Dochts (2) enthält, wobei zwischen dem Teil des Dochts und dem röhrenförmigen Rohr (5) eine Umfangskammer (11) um den Docht herum gebildet ist, so dass in der Umfangskammer (11) ein Kamineffekt erzeugt werden kann,
und einem Gehäuse (7):
wobei das röhrenförmige Rohr (5) mindestens eine seitliche Öffnung (6) aufweist, die derart angeordnet ist, dass sie den Eintritt warmer Luft von den Heizeinrichtungen (5) in die Umfangskammer (11) und deren Aufsteigen durch das Innere des röhrenförmigen Rohrs (5) zur Beeinflussung des Dochts (2) ermöglicht, und wobei die Heizeinrichtungen fixiert sind und das röhrenförmige Rohr (5) in das Gehäuse (7) derart eingesetzt ist, dass das röhrenförmige Rohr in einer Ebene und um sein Achse drehbar ist, und dass das röhrenförmige Rohr (5) nicht vertikal bewegbar ist,
und wobei die Heizeinrichtungen (4) und die Öffnung (6) des Rohres auf der gleichen Höhe angeordnet sind, so dass ein Teil des Dochts den Heizeinrichtungen durch die Öffnung (6) hindurch zugewandt sein kann.

2. Vorrichtung nach Anspruch 1, **dadurch gekennzeichnet, dass** ein Drehen des Rohres die Position der Öffnung (6) in Bezug auf die Heizeinrichtungen (4) verändert, so dass die den Docht beeinflussende Wärmemenge variiert wird.

3. Vorrichtung nach einem der vorhergehenden Ansprüche, **dadurch gekennzeichnet, dass** die Heizeinrichtungen mindestens einen nahe dem Docht angeordneten elektrischen Widerstand aufweisen.

4. Vorrichtung nach einem der vorhergehenden Ansprüche, **dadurch gekennzeichnet, dass** die Heizeinrichtungen zwei diametral zum Rohr angeordnete Widerstände aufweisen, und dass das Rohr zwei seitliche Öffnungen aufweist.

5. Vorrichtung nach Anspruch 3 oder 4, **dadurch gekennzeichnet, dass** der Widerstand oder die Widerstände mindestens eine ebene Fläche bilden.

6. Vorrichtung nach einem der Ansprüche 3 bis 5, **dadurch gekennzeichnet, dass** der Widerstand oder die Widerstände eine prismatische Rechteckform aufweisen.

7. Vorrichtung nach Anspruch 1, **dadurch gekennzeichnet, dass** bei der Drehverschiebung des Rohres eine erste Minimalverdunstungs-Endposition, in welcher die Öffnung den Heizeinrichtungen nicht zugewandt ist, und eine zweite Maximalverdunstungs-Endposition definiert sind, in welcher die Öffnung den Heizeinrichtungen zugewandt ist.

8. Vorrichtung nach einem der vorhergehenden Ansprüche, **dadurch gekennzeichnet, dass** die Heizeinrichtungen durch das Gehäuse gestützt sind.

9. Vorrichtung nach Anspruch 8, **dadurch gekennzeichnet, dass** das obere Ende des Rohres aus der Oberseite des Gehäuses ragt und einen ringförmigen Vorsprung (9) bildet, welcher das manuelle Bewegen des Rohres in Bezug auf das Gehäuse (7) erleichtert.

10. Vorrichtung nach Anspruch 8 oder 9, **dadurch gekennzeichnet, dass** das Rohr eine Umfangslippe aufweist, und dass ein Teil des Gehäuses zwischen der Umfangslippe und dem ringförmigen Vorsprung (9) angeordnet ist, wodurch ein vertikales Verschieben des Rohres verhindert, das Drehen desselben jedoch ermöglicht ist.

11. Vorrichtung nach einem der vorhergehenden Ansprüche, **dadurch gekennzeichnet, dass** das Rohr zylindrisch ist.

12. Vorrichtung nach einem der vorhergehenden Ansprüche, **dadurch gekennzeichnet, dass** sie eine Flasche (3) aufweist, welche eine aromatische Substanz und/oder eine flüchtige insektizide Substanz enthält, und wobei der Docht in die Flasche eingeführt ist.

13. Verfahren zum Verdunsten flüchtiger Substanzen unter Verwendung einer Vorrichtung nach einem der Ansprüche 1-12, wobei das Verfahren den Schritt des Umschließens eines Dochts (2) in einer kleinvolumigen Kammer (11) aufweist, welche durch ein röhrenförmiges Rohr (5) mit offenen Enden gebildet ist, so dass ein den Docht umgebender schmaler Raum zwischen dem röhrenförmigen Rohr (5) und dem Docht gebildet wird, durch welchen warme Luft aufsteigen kann, und derart, dass ein Kamineffekt in der Kammer erzeugt werden kann, wobei warme Luft durch eine in dem röhrenförmigen Rohr (5) vorgesehene seitliche Öffnung (6) eingeleitet wird, so dass sich die warme Luft durch das Innere der Kammer ausbreiten kann, wobei sie beim Aufsteigen durch das Rohr in unmittelbarer Nähe des Dochts bleibt, und wobei die Menge der in die Kammer (11) eintretenden Luft durch Drehen des röhrenförmigen Rohres (5) um dessen Achse und in einer Ebene, das heißt, ohne das röhrenförmige Rohr vertikal zu verschieben, geregelt wird, so dass der Abstand zwischen der seitlichen Öffnung (6) und der Wärmequelle verändert wird.

## Revendications

1. Dispositif pour l'évacuation de substances volatiles comprenant
des moyens de chauffage (4),
une mèche (2) à travers laquelle ladite substance remonte par capillarité, qui est influencée par lesdits moyens de chauffage (4) pour faciliter l'évaporation de ladite substance,
un tube tubulaire (5) avec des extrémités ouvertes qui contient une partie de la mèche (2), où une chambre périmétrique (11) autour de la mèche est définie entre ladite partie de la mèche et le tube tubulaire (5) de sorte qu'un effet de cheminée peut être crée dans ladite chambre périmétrique (11),
et un boîtier (7);
où ledit tube tubulaire (5) possède au moins une ouverture latérale (6) agencée pour permettre à l'air chaud desdits moyens de chauffage (4) d'entrer dans ladite chambre périmétrique (11) et de remonter à travers l'intérieur du tube tubulaire (5) pour agir sur la mèche (2), et où les moyens de chauffage sont fixés, et le tube tubulaire (5) est inséré dans le boîtier (7) de telle sorte que le tube tubulaire peut tourner dans un plan et autour de son axe, et de façon que le tube tubulaire (5) ne puisse pas être déplacé verticalement,
et où les moyens de chauffage (4) et l'ouverture (6) du tube se situent à la même hauteur de telle sorte qu'une partie de la mèche peut faire face aux moyens de chauffage à travers ladite ouverture (6).

2. Dispositif selon la revendication 1, **caractérisé en ce que** la rotation du tube change la position de l'ouverture (6) relativement aux moyens de chauffage (4) de telle sorte que la quantité de chaleur qui agit sur la mèche est modifiée.

3. Dispositif selon l'une quelconque des revendications précédentes, **caractérisé en ce que** les moyens de chauffage comprennent, au moins, une résistance électrique à proximité de la mèche.

4. Dispositif selon l'une quelconque des revendications précédentes, **caractérisé en ce que** les moyens de chauffage comprennent deux résistances positionnées diamétralement au tube, et **en ce que** ledit tube possède deux ouvertures latérales.

5. Dispositif selon la revendication 3 ou 4, **caractérisé en ce que** la ou les résistances définissent au moins une surface plate.

6. Dispositif selon l'une quelconque des revendications 3 à 5, **caractérisé en ce que** la ou les résistances ont une forme prismatique rectangulaire.

7. Dispositif selon la revendication 1, **caractérisé en ce que** dans le déplacement de rotation du tube, une première position d'évaporation minimum extrême est définie dans laquelle l'ouverture n'est pas orientée vers les moyens de chauffage, et une deuxième position d'évaporation maximum extrême dans laquelle l'ouverture fait face à ces moyens de chauffage.

8. Dispositif selon l'une quelconque des revendications précédentes, **caractérisé en ce que** les moyens de chauffage sont supportés par ledit boîtier.

9. Dispositif selon la revendication 8, **caractérisé en ce que** l'extrémité supérieure du tube fait saillie du dessus du boîtier en formant une protubérance annulaire (9) qui facilite son mouvement manuel relativement au boîtier (7).

10. Dispositif selon l'une quelconque des revendications 8 ou 9, **caractérisé en ce que** le tube possède une lèvre périmétrique, et **en ce qu'**une partie du boîtier se situe entre ladite lèvre périmétrique et la protubérance annulaire (9) en empêchant un déplacement vertical du tube mais en lui permettant de tourner.

11. Dispositif selon l'une quelconque des revendications précédentes, **caractérisé en ce que** le tube est cylindrique.

12. Dispositif selon l'une quelconque des revendications précédentes, **caractérisé en ce qu'**il comprend un flacon (3) qui contient une substance aromatique et/ou une substance insecticide volatile, et où une partie de la mèche est insérée dans ledit flacon.

13. Procédé d'évaporation de substances volatiles en utilisant un dispositif selon l'une quelconque des revendications 1 à 12, ledit procédé comprenant l'étape consistant à renfermer une portion d'une mèche (2) dans une chambre (11) d'un petit volume définie par un tube tubulaire (5) à extrémités ouvertes de sorte qu'un espace étroit est défini entourant la mèche entre ledit tube tubulaire (5) et la mèche à travers lequel l'air chaud peut remonter, et de telle manière qu'un effet de cheminée peut être crée dans ladite chambre, où l'air chaud est introduit dans ladite chambre à travers une ouverture latérale (6) réalisée dans le tube tubulaire (5) de sorte que l'air chaud se répand dans l'intérieur de la chambre en restant à proximité étroite de la mèche pendant qu'il remonte à travers le tube, et où la quantité d'air entrant dans la chambre (11) est commandée en faisant tourner le tube tubulaire (5) autour de son axe et dans un plan, c'est-à-dire sans déplacer le tube tubulaire verticalement, de sorte que la distance entre l'ouverture latérale (6) et la source de chaleur est modifiée.
